# EUROPEAN PATENT APPLICATION

(11) **EP 0 605 368 A1**
(43) Date of publication of application: **06.07.1994**
(21) Application number: 93830477.1
(22) Date of filing: 30.11.1993
(51) Int. Cl.: A61F 2/34

(54) **Prosthesis cotyl cup element for reconstructing a hip acetabulum in a hip full recovery operation**

(30) Priority: 18.12.1992 IT MI922913
(71) Applicant: ITALPRO S.p.A., I-20156 Milano (IT)
(72) Inventor: Giacometti Ceroni, Roberto, c/o ITALPRO S.p.A., I-20156 Milano (IT)
(74) Representative: Di Francesco, Gianni

(57) **Abstract**

The present invention relates to prosthesis cotyl cup element (1) for reconstructing a hip acetabulum in a hip full recovery operation, for the head portion of a prosthesis stem, comprising a cup element, including a substantially hemispheric recess (2), delimited by a circular flange (3).

The main feature of the invention is that on the flange there are provided a plurality of recesses (10) for engaging therein radially extending elements (20) which can be affixed, by screws, to sound regions of the pelvis of a patient.

## Description

### BACKGROUND OF THE INVENTION

The present invention relates to a prosthesis cotyl cup element for reconstructing a hip acetabulum in a hip full recovery operation.

As is known, in the case of fractures or deseases of the hip bone it is necessary to resect the neck of the femur, in order to apply to the body of the femur bone a prosthesis stem.

In such a type of operation it may be necessary to apply, to the patient pelvis, an acetabulum component, comprising a cup element provided for mechanically cooperating with a spheric head applied to the prosthesis stem. This operation allows to recover the articulation pivot region of the hip.

The cup element, or acetabular component, after a given time, must be frequently removed and replaced by a new acetabulum, for example because of wear, infections or other reasons.

In such a case, the operating surgeon may encounter bone lysis phenomena, which can not be anticipated a priori.

Thus, the surgeon will encounter great operation difficulties, since by the known method it is not possible to anchor a new cup element to the patient pelvis.

### SUMMARY OF THE INVENTION

Accordingly, the aim of the present invention is to overcome the above mentioned drawbacks, by providing a prosthesis cotyl construction, for reconstructing the acetabulum for the head portion of a prosthesis stem, which can be affixed to the pelvis of a patient even in the case in which the pelvis is affected by bone lysis and, accordingly, the region at which the cup element must be applied is damaged.

Within the above mentioned aim, a main object of the present invention is to provide such a cotyl construction in which the cup element can be affixed, during the surgycal operation, by additional means which can be selected by the surgeon depending on the bone conditions of the patient.

Another object of the present invention is to provide such a prosthesis cotyl construction which is very flexible in operation and which, moreover, is very safe and reliable.

According to one aspect of the present invention, the above mentioned aim and objects, as well as yet other objects, which will become more apparent hereinafter, are achieved by a prosthesis cotyl construction for reconstructing an acetabulum for a head of a prosthesis stem, comprising a cup element including a substantially hemispheric recess delimited by a circular flange, characterized in that said cotyl construction further comprises, on said flange, a plurality of recesses for engaging therein radially extending elements which can be affixed by screws to sound regions of a patient pelvis.

### BRIEF DESCRIPTION OF THE DRAWINGS

Further characteristics and advantages of the invention will become more apparent from the following detailed disclosure of a preferred, though not exclusive, embodiment of a prosthesis cotyl construction, for recovering or reconstructing an acetabulym for a prosthesis stem head, which is illustrated, by way of an indicative, but not limitative, example, in the accompanying drawings where:
Figure 1 is a schematic exploded view illustrating the subject prosthesis cotyl construction and some radially extending elements which can be associated with the cup element of the cotyl construction;
Figure 2 is a perspective view illustrating a possible embodiment of the radially extending elements;

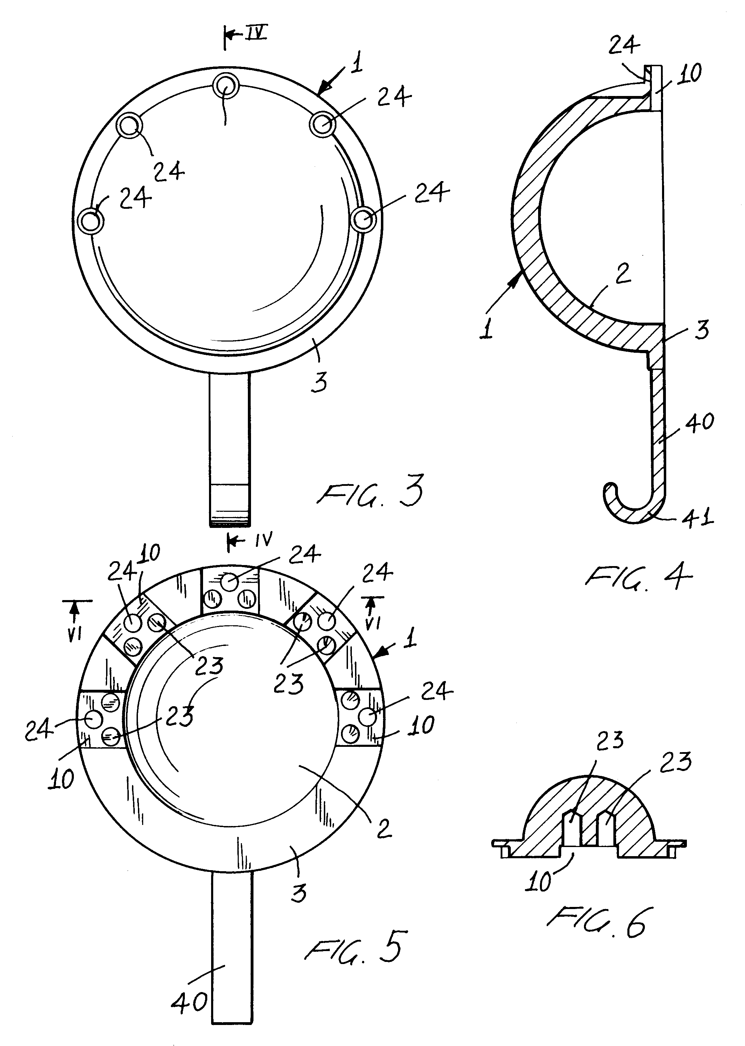

Figure 3 illustrates the cup element, as seen from its rear face;
Figure 4 is a cross-sectional view substantially taken along the line IV-IV of Figure 3;
Figure 5 shows the cup element as seen from the front face thereof;

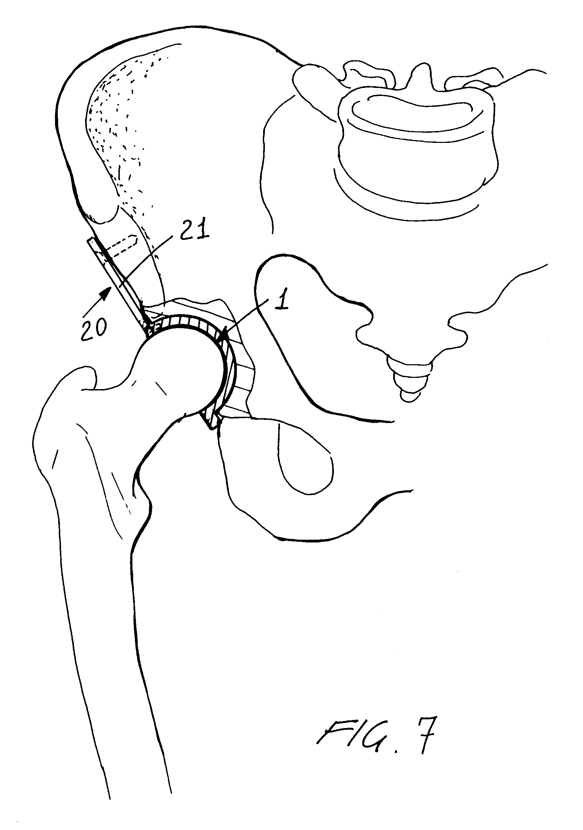

Figure 6 is a further cross-sectional view substantially taken along the line VI-VI of Figure 5;
Figure 7 is a schematic view illustrating the prosthesis cotyl construction as applied to the pelvis of a patient.

### DESCRIPTION OF THE PREFERRED EMBODIMENT

With reference to the number references of the above mentioned figures, the prosthesis cotyl construction, for reconstructing the acetabulum for the head of a prosthesis stem, according to the present invention, comprises a cup element, generally indicated at the reference number 1, which is provided with a substantially hemispheric recess 2, delimited by a circular flange 3.

As shown, the recess 2 is so designed as to be adapted to receive therein an insert made of polyethylene or any other suitable material and mechanically engageable with the head portion of a prosthesis stem, in turn engaged in the femur bone.

A main feature of the present invention is that on the flange 3 there are provided a plurality of recesses 10, for engaging therein radially extending elements, generally indicated at the reference number 20, which are provided for allowing the surgeon to anchor or affix a plurality of screws to the patient pelvis at a spaced bone region which is considered suitable in those cases in which, because of bone lysis phenomena, the bone region near the zone at which the cup element 1 must be applied is remarkably damaged.

Thus, the main operating advantage of the present invention is that the surgeon can use suitable radial anchoring elements, which can be affixed to the cup element by screws, resilient restraining elements, high strength glue materials, nails and the like, independently from the specific shape of the radial anchoring element which is used.

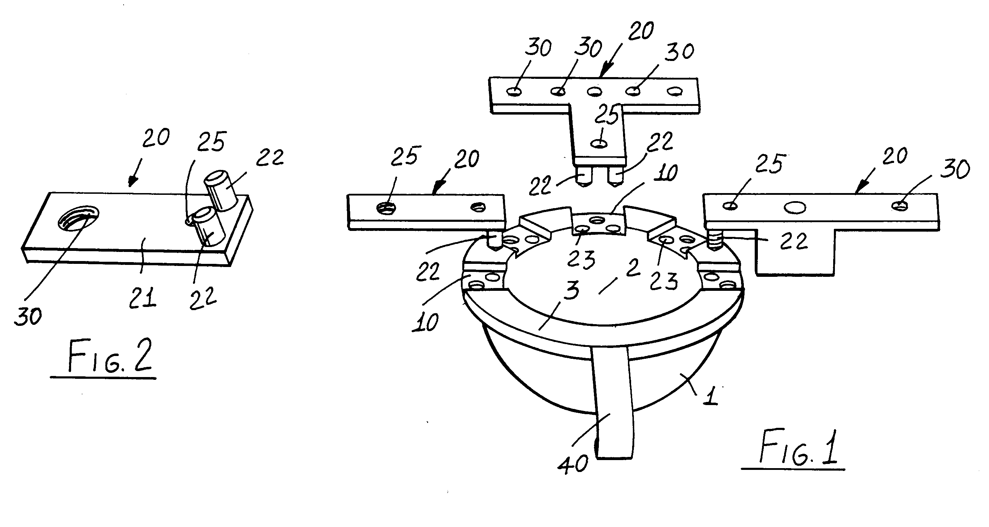

By way of an example, figure 2 shows a radial element, indicated at the reference number 20, which substantially comprises a small plate 21 including a pair of restraining pins 22, provided for engaging in blind holes 23 formed in the recesses 10.

Moreover, the recesses 10 are also provided with a tapering hole 24 arranged so as to register with a threaded hole 25 for connecting an affixing screw to the cup element.

The small plate 21, moreover, comprises a throughgoing hole, indicated at the reference number 30, in which a bone screw will be engaged for coupling with a sound bone region not affected by lisis.

From the above disclosure it should be apparent that the invention fully achieves the intended aim and objects.

More specifically, it is to be pointed out that a prosthesis cotyl construction has been provided, including a cup element which can be associated with several anchoring elements including small plates, fins or tongues and the like, which radially extend and the shape, number and arrangement whereof can be selected by the surgeon depending on the specific requirements.

To the foregoing it is to be added that from the above mentioned flange there extends a lug 40 which ends with an upturned portion 41.

The invention, as disclosed, is susceptible to several variations and modifications, all of which will come within the scope of the invention.

Moreover, all the constructional details can be replaced by other technically equivalent elements.

In practicing the invention, the used materials, provided that they are compatible to the specific use, as well as the contingent size and shapes can be any according to requirements.

## Claims

1. A prosthesis cotyl construction for reconstructing an acetabulum for a head of a prosthesis stem, comprising a cup element including a substantially hemispheric recess delimited by a circular flange, characterized in that said cotyl construction further comprises, on said flange, a plurality of recesses for engaging therein radially extending elements which can be affixed by screws to sound regions of a patient pelvis.

2. A prosthesis cotyl construction, according to Claim 1, characterized in that said recesses are evenly distributed on the substantially circular flange.

3. A prosthesis cotyl construction according to Claims 1 and 2, characterized in that it further comprises a lug extending from said flange, being rigid therewith, and ending with an upturned portion.

4. A prosthesis cotyl construction according to one or more of the preceding claims, characterized in that said recesses are provided with a pair of blind holes and a tapering throughgoing hole for engaging respectively therein a pair of restraining pins, formed on the respective radially extending elements, and with a threaded hole, for engaging therein an anchoring screw.

5. A prosthesis cotyl construction according to one or more of the preceding claims, characterized in that the number and shape of said radially extending elements are selected by the surgeon depending on requirements.

6. A prosthesis cotyl construction according to one or more of the preceding claims, characterized in that said radially extending elements are engaged in said recesses by affixing elements comprising screws, fixed-joint elements, resilient restraining elements, interference connecting elements, high resistance glue materials, nails and the like.
